# EUROPEAN PATENT SPECIFICATION

(11) **EP 1 194 174 B1**
(45) Date of publication and mention of the grant of the patent: **21.09.2005**
(21) Application number: 00940098.7
(22) Date of filing: 22.06.2000
(51) Int. Cl.: A61K 51/08, A61K 47/48, A61K 31/7088, A61K 31/711

(54) **EXTENDING THE LIFETIME OF ANTICOAGULANT OLIGODEOXYNUCLEOTIDE APTAMERS IN BLOOD**
LEBENSVERLÄNGERUNG VON ANTIKOAGULANT OLIGONUKLEOTIDE APTAMERE IM BLUT
PROLONGEMENT DE LA DUREE DE VIE D'APTAMERES D'OLIGODEOXYNUCLEOTIDES ANTICOAGULANTS DANS LE SANG

(30) Priority: 22.06.1999 US 139896 P
(43) Date of publication of application: 10.04.2002
(73) Proprietor: The University Of Alberta, Simon Fraser Uni., The Uni. of Victoria, And The Uni. Of British Columbia doing business as TRIUMF, British Columbia V7T 2A3 (CA)
(72) Inventor: DOUGAN, Alfred, H., Vancouver, British Columbia V7T 2A3 (CA); WEITZ, Jeffrey, I., Vancouver, British Columbia V6T 2A3 (CA)
(74) Representative: Vossius & Partner
(86) International application number: PCT/CA2000/000751
(87) International publication number: WO 2000/078364

(56) References cited:
- WO-A-96/40159
- US-A- 5 688 670
- DOUGAN, H. ET AL: "Extending the lifetime of anticoagulant oligodeoxynucleotide aptamers in blood" NUCL. MED. BIOL. (2000), 27(3), 289-297 , April 2000 (2000-04), XP004205326
- BOCK L C ET AL: "SELECTION OF SINGLE-STRANDED DNA MOLECULES THAT BIND AND INHIBIT HUMAN THROMBIN" NATURE,GB,MACMILLAN JOURNALS LTD. LONDON, vol. 355, no. 6360, 6 February 1992 (1992-02-06), pages 564-566, XP000453533 ISSN: 0028-0836
- BOADO, RUBEN J. ET AL: "Complete protection of antisense oligonucleotides against serum nuclease degradation by an avidin -biotin system" BIOCONJUGATE CHEM. (1992), 3(6), 519-23 , XP002161274 cited in the application
- DATABASE BIOSIS [Online] BIOSCIENCES INFORMATION SERVICE, PHILADELPHIA, PA, US; DOUGAN, H. (1) ET AL: "Radiohalogenated DNA aptamers." retrieved from STN XP002161282 & JOURNAL OF LABELLED COMPOUNDS AND RADIOPHARMACEUTICALS, (1995) VOL. 37, NO. 0, PP. 324-325. MEETING INFO.: ELEVENTH INTERNATIONAL SYMPOSIUM ON RADIOPHARMACEUTICAL CHEMISTRY VANCOUVER, BRITISH COLUMBIA, CANADA AUGUST 13-17, 1995 ,
- DOUGAN, HAYES ET AL: "Synthesis and radio-iodination of a stannyl oligodeoxyribonucleotide" NUCLEIC ACIDS RES. (1997), 25(14), 2897-2901 , XP000982488

## Description

### FIELD OF THE INVENTION

The present invention relates to compositions comprising polynucleotides that specifically bind to thrombin and to methods for extending the half-life of such polynucleotides *in vivo*. The compositions are complexes, either covalent or non-covalent, of the polynucleotide and streptavidin. The complexes include the 3' end or both ends of the polynucleotide.

### BACKGROUND OF THE INVENTION

Aptamers are nucleic acid sequences that specifically bind to target proteins and other biomolecules (16,30). Aptamers are of interest for imaging, therapy and radiotherapy applications, and they are a unique way to use nucleic acids.

### SUMMARY OF THE INVENTION

The present invention relates to complexes of DNA-aptamers with streptavidin Complexation of the aptamer with streptavidin greatly increases the half-life of the aptamer *in vivo.* The complexes can be non-covalent or covalent. Non-covalent complexes can be formed by covalent attachment of a ligand to the nucleic acid and then complexing the ligand with its specific binding protein.

The aptamer specifically binds to thrombin. Such aptamers be labeled for detection outside the body and then can be used in methods for imaging blood clots (thrombi) *in vivo*.

One problem with such methods is that the half-life of aptamers in serum is typically very short. By complexing the aptamer with streptavidin the half-life of the aptamer in serum is greatly increased. Thus, the present invention provides *in vitro* methods for imaging thrombi.

### BRIEF DESCRIPTION OF THE DRAWINGS

Figure 1: Schematic representation of complexes between thrombin (IIa) and aptamer (1A), 3'-biotinylated aptamer (1B), and 3'-biotin-streptavidin aptamer (1C). The diagram suggests why modified aptamer nucleotides can diminish affinity, while the 3'-bioconjugate is acceptable.
Figure 2: HPLC radiochromatogram of the product [¹²³I]ODN 2c (180 µCi) following purification of the radioiodinated product through a Sephadex™ G25 column in sodium phosphate (pH 7.0, 0.05M). Reverse phase HPLC employed CH₃CN in TEAB, 1 mL/min.
Figure 3: The ¹²⁵I %dose in total mouse blood is shown following injection of ODN 1b, ODN 1c, and ODN 1d (1µCi each). Mouse blood was not corrected for DNA content.
Figure 4: The ¹²⁵I %dose in total mouse blood is shown following injection of ODN 2b, ODN 2c, and ODN 2d (1µCi each). Mouse blood was not corrected for DNA content.
Figure 5: The [¹²³I]DNA %dose in total rabbit blood is shown following injection of 200 µCi ODN 1b, ODN 1c, and ODN 1d (200µCi each).
Figure 6: The [¹²³I]DNA %dose in total rabbit blood is shown following injection of 200 µCi ODN 2b, ODN 2c, and ODN 2d (200µCi each).
Figure 7: HPLC radiochromatogram of the metabolites IVDU, IVU, and iodide recovered from rabbit blood 10 min following the injection of [¹²³I] ODN 1b (3.5 mCi). Preparative and HPLC details are given in the legend of Table 5. The tracing of the ¹²³I monitor is shown. IVDU and IVU were identified by co-migration with standards.
Figure 8: *In situ* binding of aptamer to fibrinogen clots. Figure 8A shows binding of ODN 1b; Figure 8b shows binding of ODN 2b.

### DETAILED DESCRIPTION OF THE INVENTION

Aptamer polynucleotides are typically single-stranded standard phosphodiester DNA (ssDNA). Close DNA analogs can also be incorporated into the aptamer as described below.

The invention relates to DNA-aptamers that bind to thrombin.

A typical aptamer discovery procedure is described below.
1) A polynucleotide comprising a randomized sequence between "arms" having constant sequence is synthesized. The arms can include restriction sites for convenient cloning and can also function as priming sites for PCR primers. The synthesis can easily be performed on commercial instruments.
2) The target protein (thrombin) is treated with the randomized polynucleotide. The target protein can be in solution and then the complexes immobilized and separated from unbound nucleic acids by use of an antibody affinity column. Alternatively, the target protein might be immobilized before treatment with the randomized polynucleotide.
3) The target protein-polynucleotide complexes are separated from the uncomplexed material and then the bound polynucleotides are separated from the target protein. The bound nucleic acid can then be characterized, but is more commonly amplified, e.g. by PCR and the binding, separation and amplification steps are repeated. In many instances, use of conditions increasingly promoting separation of the nucleic acid from the target protein, e.g. higher salt concentration, in the binding buffer used in step 2) in subsequent iterations, results in identification of polynucleotides having increasingly high affinity for the target protein.
4) The nucleic acids showing high affinity for the target proteins are isolated and characterized. This is typically accomplished by cloning the nucleic acids using restriction sites incorporated into the arms, and then sequencing the cloned nucleic acid.

The affinity of aptamers for their target proteins is typically in the nanomolar range, but can be as low as the picomolar range. That is K_{d} is typically 1 pM to 500 nM, more typically from 1 pM to 100 nM. Apatmers having an affinity of K_{d} in the range of 1 pM to 10 nM are also useful.

Aptamer polynucleotides can be synthesized on a commercially available nucleic acid synthesizer by methods known in the art. The product can be purified by size selection or chromatographic methods.

Aptamer polynucleotides are typically from 10 to 200 nucleotides long, more typically from 10 to 100 nucleotides long, still more typically from 10 to 50 nucleotides long and yet more typically from 10 to 25 nucleotides long. A preferred range of length is from 25 to 50 nucleotides.

The aptamer sequences can be chosen as a desired sequence, or random or partially random populations of sequences can be made and then selected for specific binding to thrombin by assay *in vitro.* Any of the typical nucleic acid-protein binding assays known in the art can be used, e.g. "Southwestern" blotting using either labeled oligonucleotide or labeled protein as the probe. See also U.S. Patent 5,445,935 for a fluorescence polarization assay of protein-nucleic acid interaction.

A major problem is to utilize aptamers in vivo. In vivo, aptamers share with natural DNA a very short lifetime in blood, estimated to be 1-2 minutes. The short lifetime is sometimes attributed to serum nuclease (10). Serum nuclease results in truncated fragments of aptamer DNA, lacking affinity for the target protein.

Two aptamers have been shown to have thrombin binding properties. Aptamer d(GGTTGGTGTGGTTGG) (ODN 1a) is directed against thrombin exosite 1, the fibrinogen binding site (5). Aptamer d(AGTCCGTGGTAGGGCAGGTTGGGGTGACT) (ODN 2a) is directed against exosite 2, the heparin binding site (29). Both ODN 1b and ODN 2b bind readily to free thrombin. Radioiodination of stannyl oligodeoxyribonucleotides has provided high specific activity [¹²³I, ¹²⁵I] aptamers for this investigation (14).

*In vivo* observations with aptamer ODN 1a suggested that aptamer is lost from the blood directly to organ extraction (18,27). The lifetime of ODN 1a in serum or blood appeared longer *in vitro* than in general circulation. Few degradation products were found in circulating blood. Introduction of a heart lung bypass apparatus into a dog increased the half-life of ODN 1a to roughly 10 minutes (11). The extraction hypothesis predicts that aptamer DNA can remain intact in the blood up until extraction, available for thrombus imaging. Rapid clearance by extraction would leave insufficient time for thrombus to be labeled, while blood nuclease could make any labeling short-lived. The present invention addresses these two obstacles to extend the lifetime of aptamer in blood.

For an aptamer to retain affinity for its target protein, the same nucleotide analogs as used in the selection experiment should be used to synthesize the aptamer for practical use. Use of natural DNA nucleotides is preferred. Introduction of nucleotide analogs that abolish the contacts between the aptamer and the target protein, resulting in decreased affinity, should be avoided.

Since the principal serum nuclease is a 3'-exonuclease, protection can be afforded by 3'-end-caps on single stranded DNA (ssDNA) (1,28). The working examples below demonstrate that such end-capping increases the half-life of aptamers in circulating blood *in vivo.*

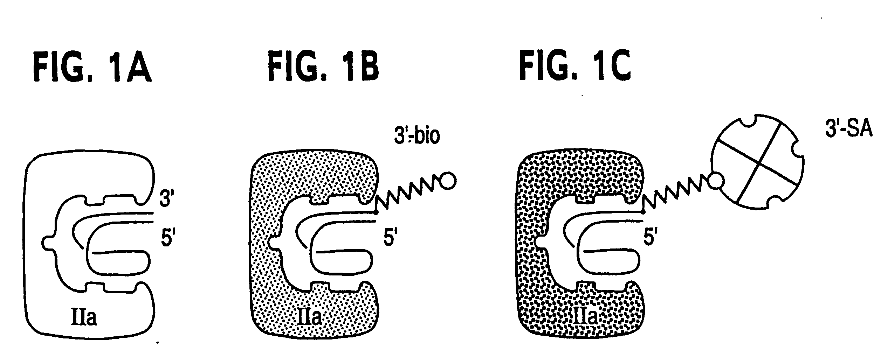

The present invention also addresses the problem of organ extraction of the aptamer. Many proteins are quite stable in circulating blood. Clearance of serum proteins by the kidney is well modeled. The working examples below also show that organ extraction may be impeded using a bioconjugate of the aptamer with a serum protein. The bioconjugate should allow the core aptamer sequence to adopt its native configuration with high affinity towards the target protein, while the protein moiety confers longer lifetime in the blood (Figure 1).

According to the invention Streptavidin (SA) is the protein for extending the half-life of an aptamer used for thrombus imaging and anticoagulation (3,25). Streptavidin has a natural half-life of 2.5 hours in the circulation of rats and rabbits. The homolog avidin is rapidly cleared from circulation. A cloned DNA encoding streptavidin and useful for making such modified proteins is disclosed in reference 31. Variant streptavidins are disclosed, for example, in reference 32.

SA can be complexed to the aptamer by derivatizing the polynucleotide at its' 3' end (preferable for use in blood) or both ends, with biotin. Methods for this reaction are known in the art. Other ligands can also be used as desired. The use of a ligand and Streptavidin for complexing the aptamer provides specificity of the site of the complexation.

Appropriate nucleotides for aptamer synthesis and their use, and reagents for covalent linkage of streptavidin to nucleic acids and their use, are considered known in the art.

The aptamer streptavidin complex of the invention can be labeled and used as a diagnostic agent *in vitro* in much the same manner as any specific protein-binding agent, e.g. a monoclonal antibody. Thus, an aptamer-streptavidin complex of the invention can be used to detect and quantitate the amount of its target protein in a sample, e.g. a blood sample, to provide diagnosis of a disease state correlated with the amount of the protein in the sample.

The working examples exemplify use of the complexes for diagnostic imaging. In imaging uses, the complexes are labeled so that they can be detected outside the body. Typical labels are radioisotopes, usually ones with short half-lives. The usual imaging radioisotopes, such as ¹²³I, ¹²⁴I, ¹²⁵I, ¹³¹I, ^{99m}Tc, ¹⁸⁶Re, ¹⁸⁸Re, ⁶⁴Cu, ⁶⁷Cu, ²¹²Bi, ²¹³Bi, ⁶⁷Ga, ⁹⁰Y, ¹¹¹In, ¹⁸F, ³H, ¹⁴C, ³⁵S or ³²P can be used. Nuclear magnetic resonance (NMR) imaging enhancers, such as gadolinium-153, can also be used to label the complex for detection by NMR. Methods and reagents for performing the labeling, either in the polynucleotide or in the protein moiety, are considered known in the art.

*In vivo*, the aptamer-protein complex of the invention can be used as an imaging agent. The working examples illustrate use of the complexes of the invention as agents for imaging thrombi *in vivo*.

For use in circulation, the complexes of the invention are formulated for intravenous administration. The formulation of proteins and nucleic acids and complexes thereof for i.v. use is considered known in the art. The dose of the complex that is administered will of course depend upon the particular utility intended.

In a therapeutic or diagnostic setting, it is considered that the dose administered can be determined by standard methods in the art. For diagnostic imaging purposes, a typical dose would be 1-10, preferably 2-5 mCi of ¹²³I compound suspended in 1 ml of physiological saline. The specific activity is preferably from 100 to 20,000 Ci/mmole, preferably 5000-20,000 Ci/mmole of the aptamer nucleic acid.

### EXAMPLES

The invention is illustrated by the following examples. The examples merely show selected embodiments of the invention and are not limiting of the invention. The scope of the invention is defined by the claims following.

### Example 1: Stabilization of a Thrombin Binding Aptamer In Vivo

### MATERIALS AND METHODS

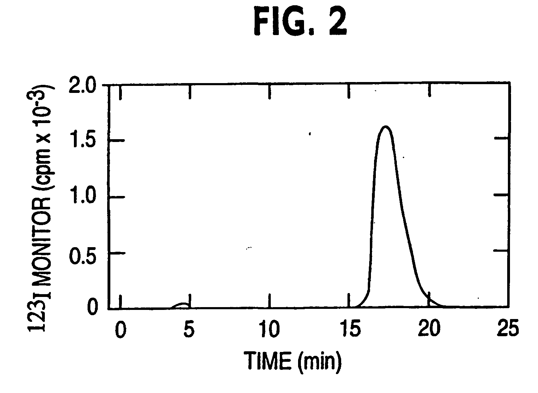

Oligodeoxynucleotides: ODNs were prepared using standard phosphoramidite chemistry by the Nucleic Acids and Protein Service laboratory at the University of British Columbia, Vancouver B.C. Stannyl ODNs and radioiodinated ODNs were prepared by our published procedure (14) (Table 1). 3'-Biotin ODN was prepared using biotin TEG CPG (Glen Research P/N: 10-1963). [¹²³I]NaI and [¹²⁵I]NaI in 0.1N NaOH were obtained from Nordion International. ODNs were prepared with specific activity 1,000-2,000 Ci/mmole (125I) or >10,000 Ci/mmole (¹²³I). Radioactive ODNs were prepared as the sodium salts, using commercial size exclusion columns, following the supplier's directions (Centri-Spin-10™, Princeton Separations (¹²⁵I); NAP-5, Pharmacia (¹²³I)). A radiochromatogram of [¹²³I] ODN 2c is given in Figure 2. Streptavidin bioconjugates of ODNs were obtained by incubating the 3'-biotin ODN in phosphate buffered saline with a fourfold excess of streptavidin (Boehringer-Mannheim) for four hours at ambient temperature. The bioconjugate was purified using a Centricon-30™ filter (Millipore) following the supplier's instructions.

Determination of aptamer affinity for thrombin: Human α-thrombin (Enzyme Research) was derivatized with fluorescein-labeled D-Phe-pro-Ala-CH₂Cl PPACK) (Haematologic Technologies) blocking the active site (6). Thrombin solution (20mM Tris, 150 mM NaCl, pH 7.4) was titrated with aptamer. The fluoresence intensity was monitored using a Luminescence Spectrophotometer (LS50b (Perkin Elmer); 492nm/522nm), and fit by non-linear regression analysis to equation 10 of reference (7) to estimate kD. Intrinsic fluorescence was measured using the setting 280nm/340nm. Carrier streptavidin led to a background of scattered light with ODNs 1f and 2f, so that duplicate measurements were performed.

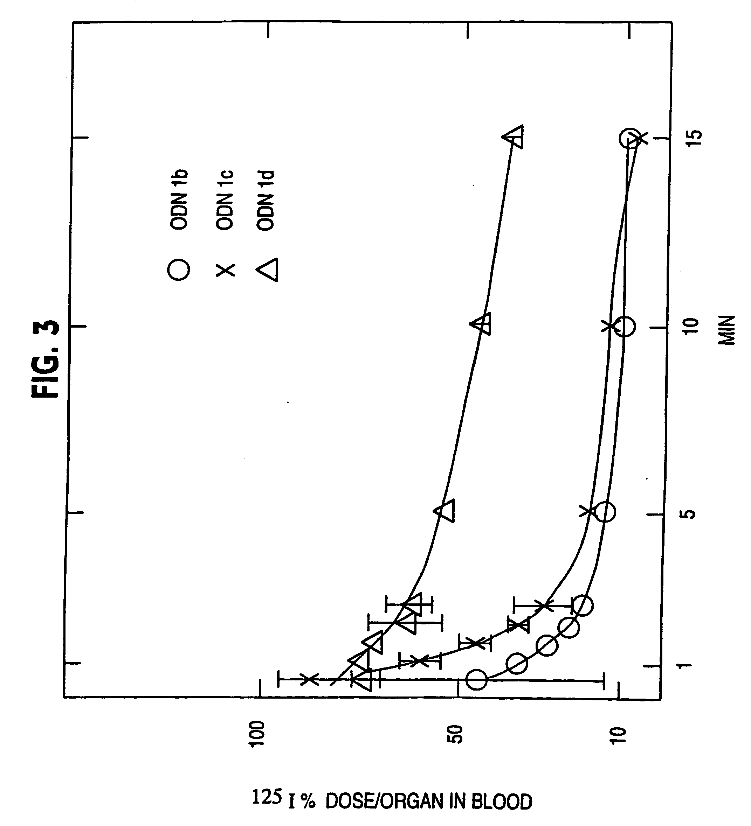

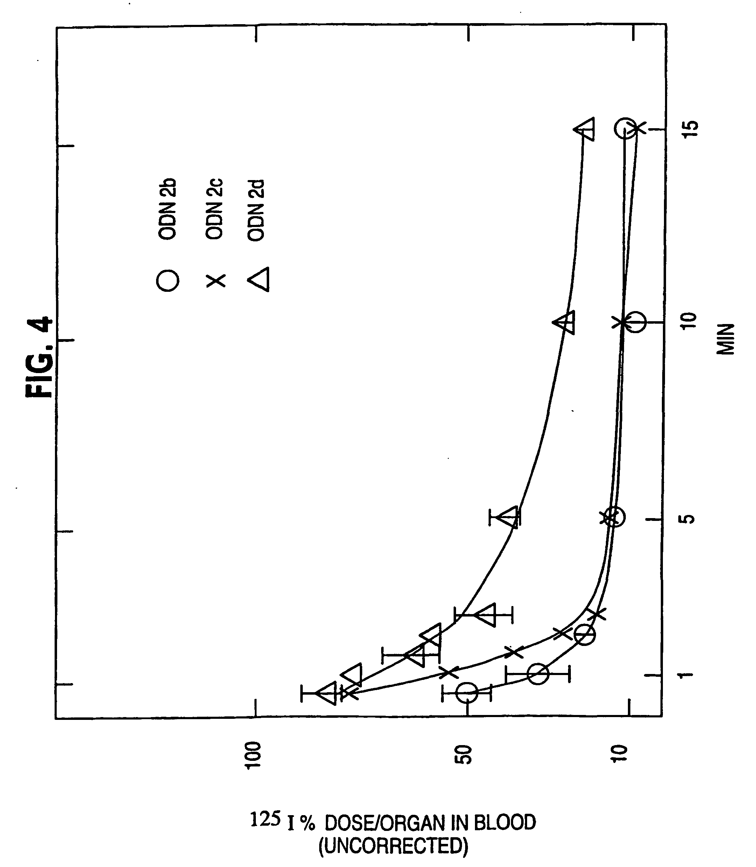

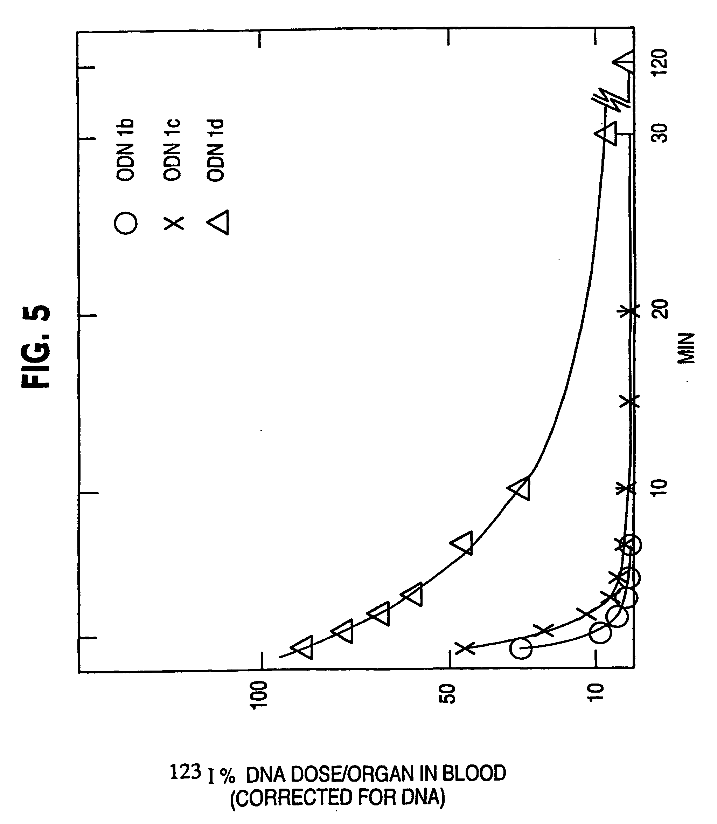

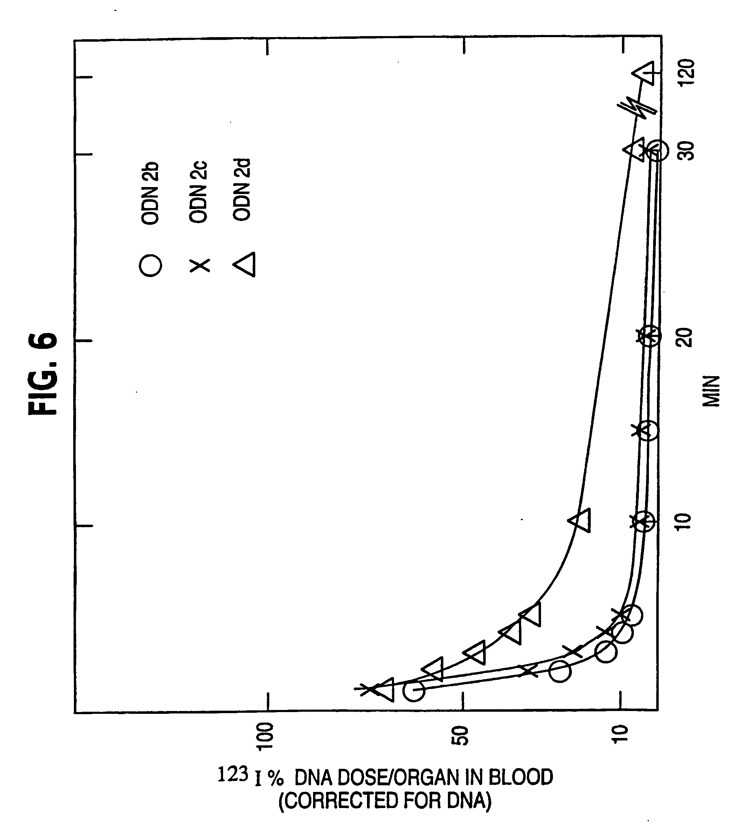

Pharmacokinetic analysis and radiobiodistribution: Six ODN compositions were investigated in animal biodistribution (Table 1). Animal procedures were approved by the UBC Committee on Animal Care, which follows the recommendations of the Canadian Council for Animal Care. Mouse: Biodistribution studies were carried out using male CDI/UBC mice (20-25g). Mice were injected in the tail vein with 37 KBq (1µCi; 0.7pmole) of [¹²⁵I] ODN in 0.1mL potassium phosphate buffer, and were sacrificed at given times post injection. The activity of the blood, liver, kidneys, spleen, stomach, lungs, heart, muscle and brain were determined and the results expressed as % injected dose per organ (Table 3A-3F, organs; Figures 3 and 4, blood). Rabbit: White female New Zealand rabbits (-3.5 kg) were used for experiments. Rabbits were anaesthetized by the i.m. injection of Ketamine hydrochloride (42 mg/kg), Acepromazine (1.0 mg/kg), and Xylazine (4.2 mg/kg). Rabbits were injected in the marginal ear vein with 0.5 mCi [¹²³I] ODN (100 pmole), and the biodistribution was estimated from scintigraphic scans. Each biodistribution is based on data from a single rabbit (Table 4A-4F). For blood studies (Figures 5 and 6) rabbits were heparinized (100 U/kg) and approximately 200µCi [¹²³I] ODN (40 pmole) was injected via ear vein; whole blood samples were taken from an artery of the opposite ear, and dispensed into pre-weighed tubes containing EDTA anticoagulant. Blood volume of the rabbit was taken to be 54 mL/kg, 1.050 g/mL (21,23). Following invasive surgical procedures, rabbits were sacrificed by the intravenous injection of Euthanyl™.

Assay of DNA bound ¹²³I: Total blood [¹²³I]DNA in the rabbit was estimated by multiplying the total blood ¹²³I (in units % dose per organ in blood) by the fraction DNA (as determined by assay) giving the result as [¹²³I]%DNA Dose/Organ in blood. Following centrifugation of blood, plasma (25 µL) was assayed by silica column (Sep Pakµ™, Waters) with elution by methanol/acetone (1/1; 2.5 mL)(15) (Figures 5 and 6). The silica Sep Pakµ™ assay was shown to retain nucleoside monophosphate and all ODNs while releasing nucleoside and free iodide. Polyacrylamide gel electrophoresis (PAGE) and silica column chromatography were adopted because trichloroacetic acid (TCA) and ethanol precipitation assays are inefficient with short oligonucleotides (<20-mer) derived from aptamers (20).

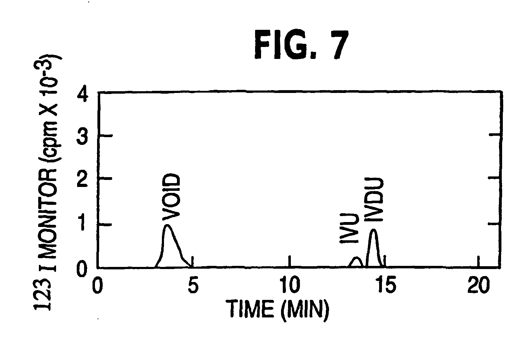

Plasma Metabolites (26): A female New Zealand rabbit (2.4 kg) was injected with [¹²³I]ODN 1b (3.5 mCi) in the marginal ear vein; plasma was obtained from blood samples (0.5 mL), and treated with an equal volume of ice cold methanol (5 min). The supernatant was evaporated, resuspended in aqueous methanol (20% vol/vol), and filtered (0.45 µm). The sample was analyzed using a C18 column (Waters: 8NVC184µ) with a linear gradient of methanol (27% to 62%, 18 min) in KH₂PO₄ (0.01M) (2 mL/min) (12,26). The ¹²³I peaks corresponded to standard 5-(2-iodovinyl)-2'-deoxyuridine (IVDU) (Prof. L. I. Wiebe) and standard [¹²⁵I]5-(2-iodovinyl)uracil (IVU), (prepared from BrVU(Sigma) (13)). It was assumed that the void represented free iodide (Figure 7; Table 5).

Digestion of ODNs by blood nuclease: [¹²⁵I] ODN (0.02 µCi, approximately 10 fmole) was suspended in 40 µL blood freshly drawn from a rabbit or mouse. The blood was incubated at simulated body temperature for a series of times (5, 10, 20, 40, 80, and 160 min). Plasma was obtained by centrifugation. Plasma (2.5 µL) was diluted in formamide (10 µL) or else urea (10 µL; urea (7M), glycerol (10%) and incubated 5 min at 95°C followed by 5 min at 0°C) and applied to a polyacrylamide sequencing-type gel (20% polyacrylamide (bisacrylamide/acrylamide(1/19)), urea (8M)) for electrophoresis (PAGE). Autoradiography was performed wich Kodak Biomax™ MS film and Biomax™ MS image intensifier (Tables 6 and 7).

### RESULTS

3'-biotin and 3'-streptavidin conjugates of the aptamers should retain affinity to thrombin(Figs 1A-1C). The affinity (kD) of biocongugate aptamers (Table 1) for fluorescein PPA-thrombin was measured by fluorometric titration, using an incident wavelength 492 nm and emission wavelength 522 nm (Table 2). Fluorescence takes place with higher characteristic quantum yield in the complex (aptamer and fluorescein PPA-thrombin) than with the free enzyme. Fluorescence measurements during titration allowed estimation of bound versus unbound thrombin as a function of aptamer molarity, and hence calculation the kD. The Ki for inhibition of coagulation by ODN 1f was verified as roughly 100nM. The kD of ODN 2f in the hydrolysis of Chromozym-thrombin was 10.2 nM.

3'-streptavidin conjugates alone (not unmodified DNA, or 3'-biotin conjugates) showed extended lifetime in blood *in vivo*. Biological evaluation was carried out with six aptamer analogs, ODNs 1b,c,d and 2b,c,d, (two bioconjugate series; Table 1). For each analog we estimated the mouse and rabbit biodistributions, with a detailed study of rabbit blood. *In vitro* blood nuclease assays supplemented the *in vivo* blood studies.

The first example, [¹²⁵I] aptamer ODN 1b, was prepared from the stannyl precursor. The biodistribution was determined in the mouse. Aliquots of aptamer (1 µCi) were injected by tail vein. Mice were sacrificed and ¹²⁵I was measured in selected organs and tissues. Results for times 0-15 min are listed in Table 3A. The ¹²⁵I increased rapidly in several organs, notably the muscle tissue, the liver and the kidney. The organ bound ¹²⁵I commenced to fall within the observation period. The lungs accumulated little ¹²⁵I. The blood ¹²⁵I %dose per organ fell rapidly up to 2.5 min (the "early phase"), and at a noticeably slower rate from 2.5 to 15 min (the "late phase") (Figures 3 and 4).

Biodistribution in the rabbit was estimated from scintigraphic images (Table 4A). Following injection of [¹²³I]ODN 1b (0.5 mCi) images were analyzed for 30 min or more. The heart, liver and kidneys were prominent. Liver uptake reached a maximum, and then diminished. Kidney uptake rapidly attained a maximum; a delay of 10 min preceded the appearance of 123I in the bladder. We found 30-40% of the injected ¹²³I in the bladder at 30-60 min post injection. The lungs accumulated little activity; the thyroids were not visible. The gall bladder did not appear in rabbit images. Two anaesthetized rabbits were injected as usual with ODN 1b (50 µCi). Samples of bile were washed from the gall bladder at 5 min intervals. Less than 0.1% of the injected dose was recovered from the gall bladder during the first 40 minutes.

Rabbit blood was analyzed separately from the image, following injection of [¹²³I] ODN 1b (200 µCi). Blood was recovered at 30 sec intervals. We observed the ¹²³I activity (%dose per organ) rise initially from 50% at 20 sec to a maximum of 66% at 30-40 sec. The ¹²³I %dose per organ then decreased rapidly, approaching 24% at 5 minutes. From ∼10 min onward, the ¹²³I activity decreased more slowly, reaching 17.8% at 30 min. The pattern of ¹²³I elimination again suggested an "early" phase and a "late" phase.

The difference between early phase and late phase blood was examined following the injection of ODN 1b into rabbits. In both phases, ¹²³I activity was located in the blood plasma following the injection of CDN. Chromatography of plasma revealed that early phase plasma ¹²³I bound to SiO₂, suggesting that the ¹²³I was incorporated into DNA. Late phase plasma ¹²³I bound roughly 3% to the SiO2, suggesting that metabolites below the nuelectide monophosphate level predominated in late phase blood (Figures 5 and 6). Total blood [¹²⁵I]DNA was estimated by multiplying the total blood ¹²³I (in units % dose per organ in blood) by the fraction DNA (as determined by SiO₂ chromatography) giving the result in the following units: [¹²³I]%DNA Dose/Organ in blood. In the case of ODN 1b, the metabolites in rabbit plasma were identified and characterized by reverse phase HPLC (Figure 2; Table 5). The major ¹²³I peaks coincided with 5-(2-iodovinyl)-2'deoxyuridine (IVDU), 5-(2-iodovinyl)uracil (IVU), and probably free iodide (i.e. the HPLC void). IVDU was the most abundant metabolite at 5 min, while iodide predominated at later times.

Rapid removal of ODN 1b from blood is evidently a serious problem. We attempted to alleviate this problem with the 3'-biotin (ODN 1c) and 3'-streptavidin bioconjugates (ODN 1d). Similar bioconjugates of the exosite 2 aptamer (ODNs 2b, c, d) were evaluated to determine whether the results were sequence-specific. A comparative analysis was extended to ODNs 1c, d and 2b, c, d (Tables 3A-F, and 4A-F):

Blood: Modified aptamers behaved in characteristic ways in the blood (Figures 3-6), depending on 3'-modification, and not depending much on the DNA sequence. Total blood radioiodine pharmacokinetics was initially measured in the mouse and rabbit. Repeated mouse results demonstrated statistical validity (i.e. acceptable standard deviation (SD)). Mouse and rabbit results resembled each other. Blood concentrations were initially high, but fell as radiotracer was removed. We have pointed out the "early phase" and "late phase" phenomenon in the blood. The initial blood radioiodine level (% dose per organ) was significantly higher for 3'-biotin aptamers (ODNs 1c and 2c) than unmodified aptamers. The "late phase" blood % dose per organ of 3'-biotin aptamers superimpose upon the levels for unmodified aptamers. Modified aptamers with 3'-SA showed elevated blood radioiodine initially and also later (compared to unmodified and 3'-biotin).

DNA was assayed directly in rabbit plasma (Figures 5 and 6). The pharmacokinetics showed prolonged lifetime of aptamer in blood, although the data suggested multicomponent kinetics. Mean lifetime was calculated by numerical integration. For ODN 1d the mean lifetime was estimated 7.9 minutes based on data from 0 min to 30 min, 15.2 min (data from 0 min to 90 min), and 18.6 min (data from 0 min to 120 min). For ODN 2d the mean lifetime was 8.7 min (data 0 min to 30 min), 23 min (data 0 min to 90 min), and 31 min (data 0 min to 120 min). Further clarification of the nature of the long-lived component would be of interest.

To supplement the blood biodistribution data, we developed a simulated in vivo nuclease degradation assay for [¹²⁵I]ODNs. In preliminary work (data not presented), we found that ODN 1b and ODN 2b were both stable in pre-frozen rabbit plasma up to 160 min at 39°C. We developed a nuclease assay in freshly drawn blood (heparinized) from mice or rabbits. At 20-23°C no digestion of ODN 1b or ODN 2b was observed during 160 min. At simulated body temperature, considerable digestion was observed. Autoradiographs showed mainly the bands from intact ODNs. Faint ladders of truncated ODNs were observed ahead of intact ODN 1b and ODN 2b. Ladders were previously reported in vivo with ODN 1a monitored by HPLC or laser stimulated fluorescence (18,24,27). Ladders were not observed ODNs 1c,d or 2c,d.

Thus, [¹²⁵I]ODNs were incubated in vitro in freshly drawn mouse blood (36°C) or rabbit blood (39°C) (Tables 6 and 7). DNA was recovered from the plasma and examined by denaturing PAGE and autoradiography. The halflife of ODNs 1b and 2b were estimated ∼10 min in mouse blood or ∼5 min in rabbit blood. The halflife of biotinylated ODN 1c was ∼160 min in mouse blood or rabbit blood, while the halflife of 2c was ∼80 min in mouse blood and ∼40 min in rabbit blood. The halflife of the streptavidin bioconjugates 1d and 2d appeared to be 160 min or greater in either mouse blood or rabbit blood.

Liver: Liver uptake showed the highest % dose per organ with nearly all the ODNs, in both the mouse and the rabbit, although uptake in the kidneys and muscle was comparable. The maximum was achieved quickly, at 1-2 min; maximal uptake of streptavidin analogs was delayed to roughly 5 min in the mouse. Liver activity decreased rapidly from the maximum. Mouse liver uptake seemed not to distinguish the pairs 1b/2b and 1c/2c; however the uptake of 2d exceeded 1d. In the rabbit, uptake of ODN 2b,c,d exceeded the corresponding ODN 1b,c,d. With mouse data we have good quantitative and statistical certainty. Mouse liver maxima showed sequence dependence 1b>1c>1d or 2b=2c<2d. Rabbit data differed slightly from this rule. We did not detect the rabbit gall bladder in images.

Kidneys: Kidney % dose per organ was as high as 15%. Broad maxima for ODNs 1b and 2b were observed at 1.0 -1.5 min. Maxima for the bioconjugates were observed at increasingly later times, with the maxima for ODNs 1d and 2d at 5 min, and of lesser magnitude. In the mouse kidney, uptake of ODN 2b,c,d exceeded the analogous ODN 1b,c,d. Rabbit images show that the bladder contained 30-40% of the injected dose following ODN 1b injection. Although the kidney/bladder route assumed significance with time, the rabbit bladder was not observed until ∼10 min postinjection of 1b,c,d or 2b,c,d. This observation suggests that the kidney uptake may represent nearly the total in the kidney/bladder pathway at early times.

Muscle: Mouse muscle showed a low % dose per gram for all ODNs; the % dose per organ assumed significance (up to 27%). Maxima were attained early by 1b or 2b (1 min), later by 1c or 2c (5 min), and still later by 1d or 2d (10-15 min).

Lungs: Mouse lungs attained the highest % dose per gram of the solid organs following the injection of the streptavidin and biotin bioconjugates. Mouse blood showed even higher % dose per gram than the lung in these instances. Lung activity may indicate physiological uptake, or it may reflect contamination of lung tissue with blood. In rabbits we did not observe lung images following the injection of [1231] bioconjugate ODN analogs (i.e. 1b,c,d or 2b,c,d).

Thyroids: Thyroids did not image during 30 min scintigraphic studies with rabbits.

The biodistributions reported here generally agree with the biodistribution of tritiated ODN la reported in rats (24). Differences may be attributed to IVDU and bioconjugates in our ODNs contrasted to natural DNA in ref (24), and species differences.

### CONCLUSIONS

Short lifetime in blood is a general obstacle to the application of phosphodiester DNA as a radiopharmaceutical, and a particular problem with DNA aptamer radiotracers. Serum nuclease, and also organ extraction have been proposed as the fundamental causes of this problem. 3'-biotin-streptavidin bioconjugates of the aptamers retain affinity and show extended lifetimes in blood.

The biodistribution studies confirmed that unprotected phosphodiester DNA aptamers are short-lived like other ODNs, whether in circulation or extracted blood. The biodistributions likely reflect the uptake of intact aptamers at early times. Aptamers showed rather long *in vitro* lifetimes by PAGE assay *in vitro* in fresh blood. Estimates were 5, 10, 40, 80 or >160 min, depending on the analog (ODN 1b,c,d or 2b,c,d) and animal species. These lifetime estimates imply that many analogs might be intact *in vivo* at 1, 5, 10 or 15 min following injection *in vivo.* Literature lifetimes for ODN 1a in serum *(in vitro)* range from 15 to 30 min. The guanine quartet structure may stabilize both ODNs 1 and 2 in serum relative to ODNs lacking secondary structure. Binding to prothrombin is an additional factor believed to extend the *in vitro* lifetime of ODN 1 analogs (11,18,27). Extended DNA levels in circulating blood with streptavidin aptamer bioconjugates also support this idea. Sequence dependent biodistribution differences between ODN 1 and 2 analogs also suggest uptake of intact DNA. The times of maximal organ uptake (especially liver, muscle, and kidneys) appear to be determined by the early phase DNA availability; certain organ maxima may occur at later times with the bioconjugates because the DNA availability is prolonged.

Total radioiodine (ODN plus metabolites) cleared from circulating blood in two stages in both mouse and rabbit. Labeled DNA aptamer predominated during the early stage. During the late stage, metabolites predominated (i.e. IVDU, IVU, and iodide). For unmodified aptamers ODNs 1b and 2b, the half-life in circulation is considered to be 1.0 to 1.5 min (11,18,24,27). This lifetime was scarcely increased in the 3'-biotinylated analogs, even though they were stable to nuclease in non-circulating blood. Prolonged mean lifetime in blood of 18.6 min or longer was obtained with the 3'-streptavidin bioconjugates.

It has been proposed that ODN la is cleared by organ extraction, especially lung extraction (11,18,27). Stabilization of aptamers by streptavidin (and not by biotin alone) suggests that ODNs might pass through a filter (analogous to the kidney glomerulus) into another compartment during natural clearance and degradation. High concentrations of ODNs 1d and 2d are found in mouse lungs (but not rabbit lungs). The above results suggest at early times the kidneys carry only 8 to 15% of the injected dose, with little in the bladder. The data indicate that the majority of ODN (85 to 92%) is extracted by organs other than the kidney; extraction models are unavailable. The multicomponent nature of the clearance kinetics suggests that more than one form of the streptavidin complex may be involved. From a biochemical standpoint, serum 3'-exonuclease is well known; 3'-end-capping of ODN is sufficient to extend the lifetime in serum (1,28). However 3'-end-capped phosphodiester ODNs are short-lived in circulating blood, suggesting that the stability problem has more components. In one example, an ODN methylphosphonate with a single 5'-phosphodiester proved stable in non-circulating blood, but truncated ODNs were recovered in circulating blood, or with organ extracts (4,9). This suggested passage of ODN back and forth between blood and solid organs. Phosphorothioate ODNs are primarily resistant to serum nuclease. They exhibit non-specific binding to serum proteins as a secondary characteristic (8). Reversing this scenario, we have bound phosphodiester ODNs to a simulated serum protein, and clearance of the ODNs was impeded.

### EXAMPLE 2: Di-biotinylated Aptamers

### MATERIALS AND METHODS

Oligodeoxynucleotides: 5'-Biotinylation was carried out with a modification of the stannyl oligonucleotide reactions (14). The 5'-Fmoc stannyl ODN on the solid support was treated with 1,8-diazabicyclo[5.4.0]undec-7-ene (DBU) (0.1M in acetonitrile, 2.5 min) to remove the Fmoc. The solid support was washed with acetonitrile, and then reacted with biotin TEG phosphoramidite (Glen Research P/N: 10-1955) according to the manufacturer's directions. The product was oxidized using 3-chloroperoxybenzoic acid, as described previously (14). The standard stannyl cleavage and deprotection procedures were carried out (14), leading to the di-biotinylated, stannyl ODN with the 5'-DMT still in place. The product ODN was purified by standard HPLC (14) using a gradient of acetonitrile in TEA bicarbonate (0.15M, pH 7.0) where a single peak was collected (tR=41 min), and divided into portions of 100µg. The ODN was radioiodinated using ¹²³I and a standard Iodobead procedure (14). The radioiodinated product (DMT-on) was resolved by standard HPLC at tR=35 min; this peak was collected and evaporated to dryness in the vacuum centrifuge. Detritylation was performed by suspending the dry product in aqueous acetic acid (80%, 50 µL) for 20 min at ambient temperature. The acetic acid was removed under vacuum, and the ODN was evaporated a second time in the presence of TEA (10 µL). The radioiodionated product was purified directly with a NAP-5 column. The product gave a single ¹²³I peak (tR=19 min) on HPLC.

Aptamer stability in blood was assessed by ¹²³I content in blood DNA in the same manner as in Example 1.

### RESULTS

Aptamer stability in blood was enhanced further (compared to 3'-biotinylated aptamer) when a doubly biotinylated aptamer (i.e. at both 3'- and 5'- ends) was conjugated with streptavidin. This would lead to a type of cyclic oligonucleotide bioconjugate with a single streptavidin tetramer, or else a linking of two streptavidin tetramers. With the di-biotin streptavidin bioconjugate a half life of roughly 90 min was found in rabbit blood.

### Example 3: In situ Binding of Aptamer to Fibrinogen Clots

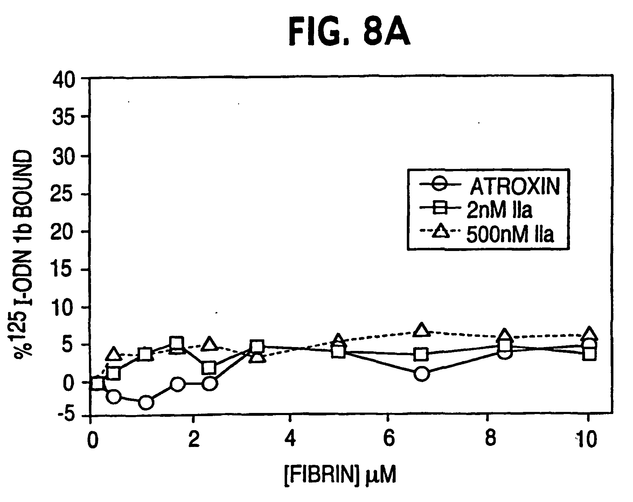

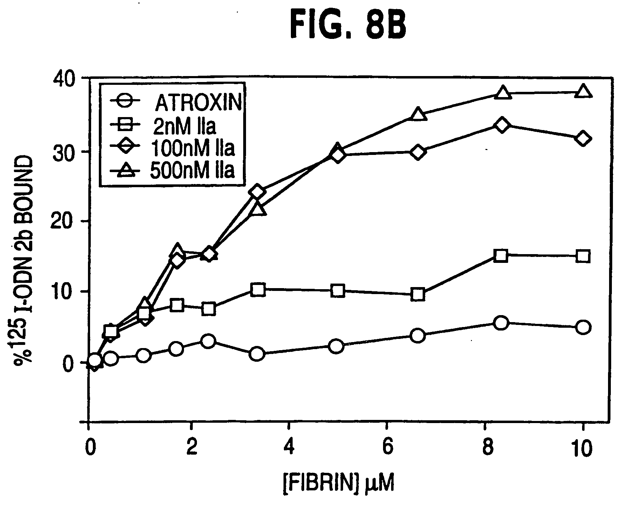

Reaction mixtures were made of TSTW (50 µl) supplemented with fibrinogen (0 to 10 µM), CaCl₂ (2 mM), [¹²⁵I]ODN (0.1 µCi, 20 nM) and IIa (0.2 to 500 nM). Atroxin replaced fibrinogen in control reactions. Following clotting, (1 hr), reaction mixtures were centrifuged. Free and bound aptamer were then determined. Results are shown in Figures 8A and 8B.

### REFERENCES

The following articles of the periodical and patent literature are cited throughout the specification. Each article is hereby incorporated by reference in its entirety by such reference.
1. Agrawal S. and Goodchild J. (1987) Oligonucleoside methylphosphonates: synthesis and enzymic degradation. Tetrahedron Lett. 28, 3539-3542.
2. Beigelman L., McSwiggen J.A., Draper K.G., et al. (1995) Chemical modification of hammerhead ribozymes. J. Biol. Chem. 270, 25702-25708.
3. Boado R.J. and Pardridge W.M. (1992) Complete protection of antisense oligonucleotides against serum nuclease degradation by an avidin-biotin system. Bioconj. Chem. 3, 519-523.
4. Boado, R.J., Kang, Y-S., Wu, D. and Pardridge, W. M. (1995) Rapid plasma clearance and metabolism in vivo of a phosphorothioate oligodeoxynucleotide with a single, internal phosphodiester bond. Drug Metab. Dispos. 23, 1297-1300.
5. Bock L.C., Griffin L.C., Latham J.A., Vermaas E.H. and Toole J.J. (1992) Selection of single-stranded DNA molecules that bind and inhibit human thrombin. Nature 355, 564-566.
6. Bock, P.E. (1992) Active-site-selective labelling of blood coagulation proteinases with fluorescence probes by the use of thioester peptide chloromethyl ketones. J. Biol. Chem. 267, 14974-14981.
7. Boskovic, D.S., Giles, A.R., and Nesheim, M.E. (1990) Studies of the role of Factor Va in the Factor Xacatalyzed activation of Prothrombin, Fragment 1.2-Prethrombin, and dansyl-L-glutamyl-glycyl-L-arginine-Meiothrombin in the absence of phospholipid. J. Biol. Chem. 265, 10497-10505.
8. Brown D.A., Kang S.H., Gryaznov S.M. et al. (1994) Effect of phosphorothioate modification of oligodeoxynucleotides on specific protein binding. J. Biol. Chem. 269, 26801-26805.
9. Chen T.L., Miller P.S., Ts'o P.O.P., and Colvin O.M. (1990) Disposition and metabolism of oligodeoxynucleoside methylphosphonate following a single iv injection in mice. Drug Metab. Dispos. 18, 815-818.
10. Crooke S.T. (1998) Cellular uptake, distribution, and metabolism of phosphorothioate, phosphodiester and methylphosphonate oligonucleotides. In: Antisense Research and Applications (Edited by Agrawal S. and Crooke S.T.). Springer, New York.
11. De Anda S., Coutre S.E., Moon M.R. et al. (1994) Pilot study of the efficacy of a thrombin inhibitor for use during pulmonary bypass. Ann. Thorac. Surg. 58, 344-350.
12. Desgranges C., Razaka G., Rabaud M., Bricaud H., Balzarini J. and De Clercq E. (1983) Phosphorolysis of E-5-(2-bromovinyl)-2'-deoxyuridine (BVDU) and other 5-substituted-2'-deoxyuridines by purified human thymidine phosphorylase and intact blood platelets. Biochem. Pharmacol. 32, 3583-3590.
13. Dougan H., Rennie B.A., Lyster D.M. and Sacks S.L. (1994) No-carrier-added [123I]1-(á-D-arabinofuranosyl)-5(E)-(2-iodovinyl)uracil (IVaraU): High yielding radiolabeling via organotin and exchange reactions. Int. J. Appl. Radiat. Isot. 45, 795-801.
14. Dougan H., Hobbs J.B., Weitz J.I. and Lyster D.M. (1997) Synthesis and radioiodination of a stannyl oligoribodeoxynucleotide. Nuc. Acids Res. 25, 2897-2901.
15. Fisher J., Johnston A.M., Holland T.K., McCallum J., Pescador R., Montovani M., and Prino G. (1993) Pharmacokinetics, absorption, distribution and disposition of [125I] defibrotide following intravenous or oral administration in the rat. Thromb. Res. 70, 77-99.
16. Gold L., Polisky B., Uhlenbeck O. and Yarus M. (1995) Diversity of oligonucleotide functions. Ann. Rev. Biochem. 64, 763-797.
17. Kubik M.F., Bell C., Fitzwater T., Watson S.R. and Tasset D.M. (1997) Isolation and characterization of 2'-fluoro, 2'-amino, and 2'-fluoro/amino modified RNA ligands to human IFN-gamma that inhibit receptor binding. J. Immunol. 159, 259-267.
18. Lee W.A., Fishback J.A., Shaw J.P., Bock L.C., Griffin L.C. and Cundy K.C. (1995) A novel oligonucleotide inhibitor of thrombin. II. Pharmacokinetics in the cynomolgus monkey. Pharm. Res. 12, 1943-1947.
19. Lin Y., Gill S.G. and Jayasena, S.D. (1994) Modified RNA sequence pools for in vitro selection. Nuc. Acids Res. 22, 5229-5234.
20. Maniatis T, Fritsch E.F. and Sambrook J. (1982) Molecular Cloning. Cold Spring Harbor Laboratory, New York. 473.
21. Manning P.J., Ringler D.H., and Newcomer C.E. (1994) Biology of the Laboratory Rabbit. Academic Press, San Diego.
22. Pieken W.A., Olsen D.B., Benseler F., Aurup H. and Eckstein F. (1991) Kinetic characterization of ribonuclease resistant 2'-modified hammerhead ribozymes. Science 253, 314-317.
23. Prince H. (1982) Blood volume in the pregnant rabbit. J. Exper. Physiol. 67, 87-95.
24. Reyderman L. and Stavachansky, S. (1998) Pharmacokinetics and biodistribution of a nucleotide-based thrombin inhibitor in rats. Pharm. Res. 15, 904-910.
25. Rosebrough S.F. (1993) Pharmacokinetics and biodistribution of radiolabeled avidin, streptavidin and biotin. Nucl. Med. Biol. 20, 663-668.
26. Samuel J., Gill M.J., Iwashina T., Tovell D.R., Tyrrell D.L., Knaus E.E. and Wiebe L.I. (1986) Pharmacokinetics and metabolism of E-5-(2-[131I]iodovinyl)-2'-deoxyuridine in dogs. Antimicrob. Agents Chemother. 29, 320-324.
27. Shaw J.P., Fishback J.A., Cundy K.C. and Lee W.A. (1995) A novel oligonucleotide inhibitor of thrombin. I. Metabolic stability in plasma and serum. Pharm. Res. 12, 1937-1942.
28. Stein C.A., Subasighe C., Shinozuka K. and Cohen J.S. (1988) Physicochemical properties of phosphorothioate oligodeoxynucleotides. Nuc. Acids Res. 16, 3209-3221.
29. Tasset D.M., Kubik M.F. and Steiner W. (1997) Oligonucleotide inhibitors of human thrombin that bind distinct epitopes. J. Mol. Biol. 272, 688-698.
30. Uphoff K.W., Bell S.D. and Ellington A.D. (1996) In vitro selection of aptamers: the dearth of pure reason. Curr. Opin. Struct. Biol. 6, 281-288.
31. Sato T, et al. (1990) Expression of a cloned streptavidin gene in Eschericia coli. Proc. Natl. Acad. Sci. 87, 1442-146.
32. Wilbur D.S. et al. (1998) Streptavidin in antibody pretargeting. Comparison of a recombinant streptavidin with two strepatvidin mutant proteins and two commercially available streptavidin proteins. Bioconjugate Chem. 9, 100-107.

### TABLES

TABLE 1: Oligodeoxynucleotide aptamers directed to human thrombin exosites 1 and 2 used in this work: a: X=Y=nil; b: X=IVDU, Y=nil; c: X=IVDU, Y=biotin; d: X=IVDU, Y=biotin-streptavidin; e: X=nil, Y=biotin; f: X=nil, Y=biotin-streptavidin.
TABLE 2: Fluorometric assay of the binding K_{d} of bioconjugate aptamers to human α-thrombin with a fluorescein-PPACK-labeled active site. The emitted intensity (522 nm) was recorded before and after the addition of aptamer to a thrombin solution (100nM). The incident wavelength was 492 nm.
TABLES 3A-F: Mice received [¹²⁵I] ODN (1 µCi) by tail vein, and were sacrificed and dissected at the given times. ¹²⁵I was assayed in the tissues and organs. Except where indicated, n=3. The identity of the ODN is indicated on each chart.
TABLES 4A-F: Anaesthetized female New Zealand rabbits received [¹²³I] ODN (0.5 mCi) by ear vein. Rabbits were laid in the supine position on the detector and images were obtained based on ten second counting frames. The biodistribution was estimated from analysis of counts in the region of interest compared to the total counts.
TABLE 5: Blood metabolites were analyzed by HPLC following the injection of [¹²³I]ODN 1b into a female New Zealand rabbit. Plasma was obtained from blood samples (0.5mL), and was treated with an equal volume of methanol at 0°C. The supernatant was evaporated, resuspended, and filtered, prior to HPLC (12,26). Reverse phase HPLC employed a gradient of methanol (27% to 62% over 18 min) in aqueous KH₂PO₄ (0.01M, 2 ml/min). IVDU and IVU were identified by migration with standards. Samples (1 mL) were collected in plastic vials for quantitative measurement in a well-type counter.
TABLE 6: [¹²⁵I] ODN was incubated in 40 æL blood freshly drawn from a mouse for times indicated at 36°C. Plasma (2.5 µL) was diluted in formamide (10 µL) or else urea (10 µL; urea (7M), glycerol (10%)) and the DNA was resolved by PAGE. The autoradiograph was scored for the presence (+) or absence (-) of full length aptamer.
TABLE 7: [¹²⁵I] ODN was incubated in 40 µL blood freshly drawn from a rabbit for times indicated at 39°C. The procedure was otherwise similar to Table 6.

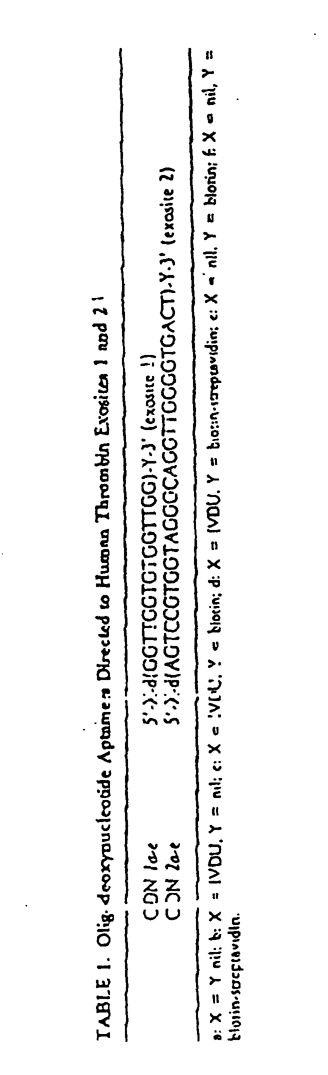

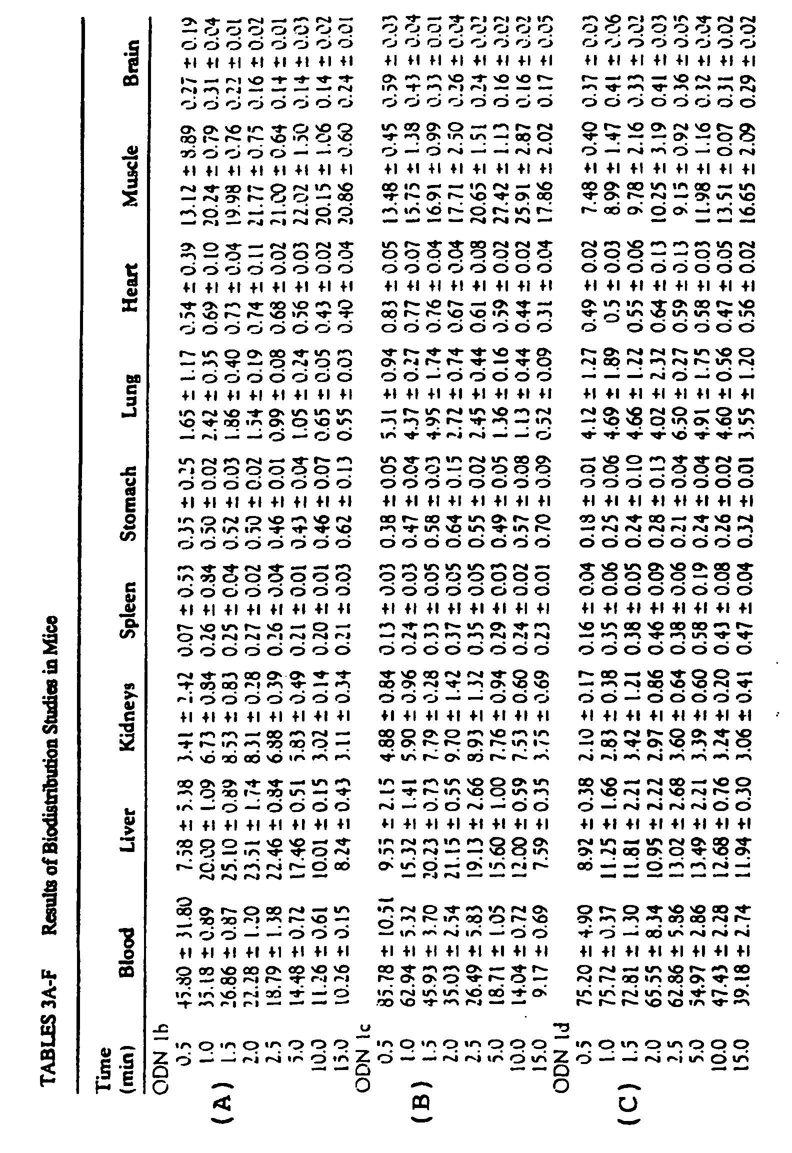

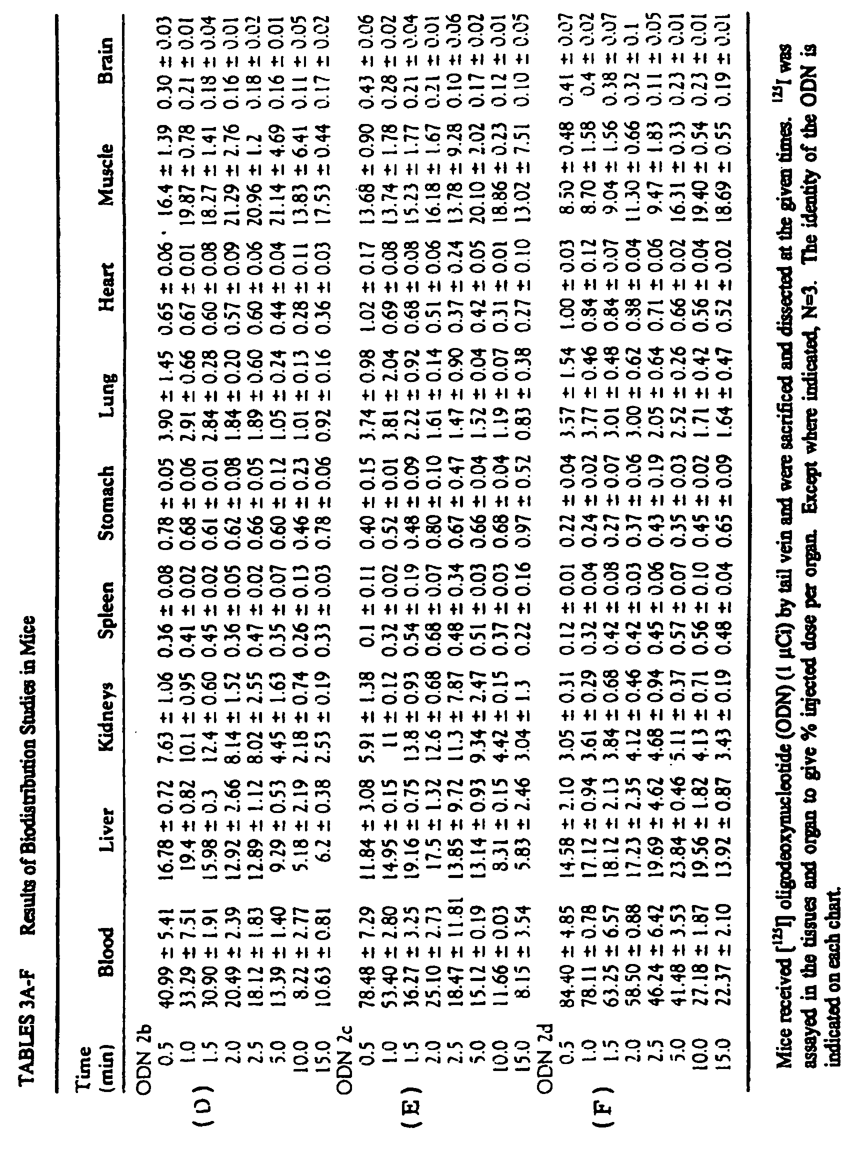

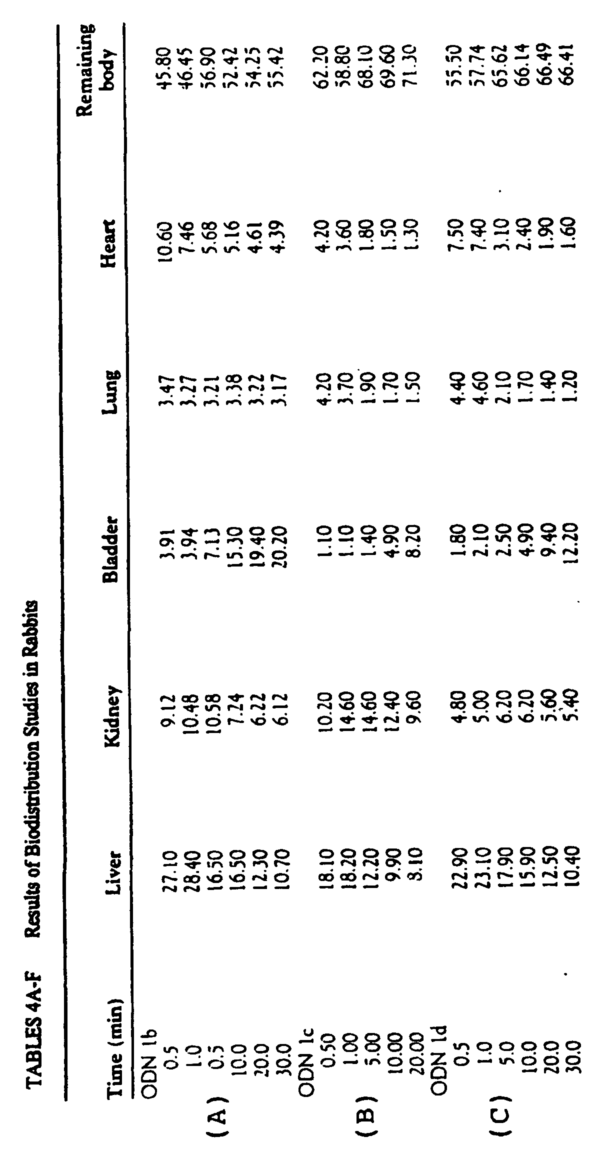

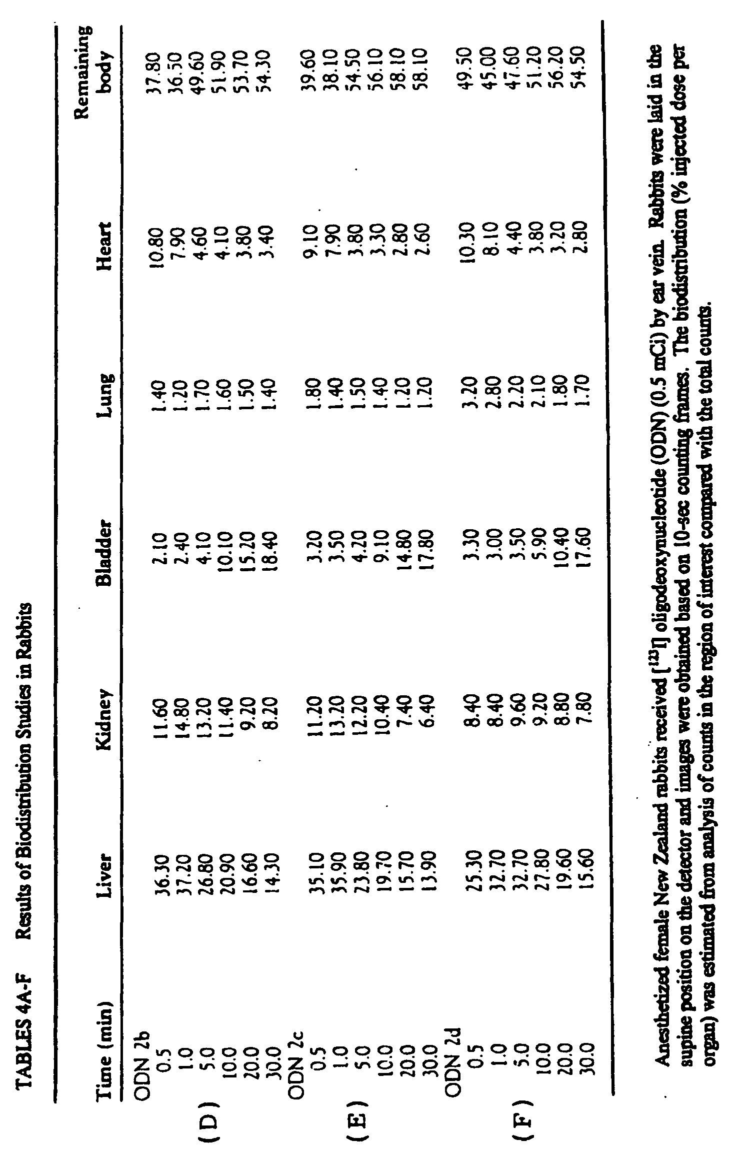

**TABLE 5:**

| **Blood Metabolites** | | | |
|---|---|---|---|
| | 5 min | 15 min | 30 min |
| IVDU | 46.7% | 23.4% | 11.6% |
| IVU | 13.2% | 7.5% | 5.0% |
| Iodide | 32.8% | 63.3% | 78.3% |
| Table 5. Blood metabolites were analyzed by HPLC following the injection of [¹²³I]ODN 1b into a female New Zealand rabbit. Plasma was obtained from blood samples (0.5mL), and was treated with an equal volume of methanol at 0°C. The supernatant was evaporated, resuspended, and filtered, prior to HPLC (12,26). Reverse phase HPLC employed a gradient of methanol (27% to 62% over 18 min) in aqueous KH₂PO₄ (0.01M, 2 ml/min). 5-(2-iodovinyl)-2'-deoxuridine (IVDU) and [¹²⁵I]5-(2-iodovinyl)uracil (IVU) were identified by migration with standards. Samples (1 mL) were collected in plastic vials for quantitative measurement in a well-type counter. | | | |

**TABLE 6:**

| **Results of Autoradiography of Mouse Blood** | | | | | | |
|---|---|---|---|---|---|---|
| ODN | 5 min | 10 min | 20 min | 40 min | 80 min | 160 min |
| ODN-1b | + | + | (+) | - | - | - |
| ODN-1c | + | + | + | + | + | + |
| ODN-1d | + | + | + | + | + | + |
| ODN-2b | + | + | (+) | - | - | - |
| ODN-2c | + | + | + | + | + | - |
| ODN-2d | + | + | + | + | + | + |
| Table 6. [¹²⁵I] oligodeoxynucleotide (ODN) was incubated in 40 µL blood freshly drawn from a mouse for times indicated at 36°C. Plasma (2.5 µL) was diluted in formamide (10 µL) or urea (10 µL; urea (7M), glycerol (10%)) and the DNA was resolved by PAGE. The autoradiograph was scored for the presence (+) or absence (-) of full length aptamer. | | | | | | |

**TABLE 7:**

| **Results of Autoradiography of Rabbit Blood** | | | | | | |
|---|---|---|---|---|---|---|
| ODN | 5 min | 10 min | 20 min | 40 min | 80 min | 160 min |
| ODN-1b | + | (+) | - | - | - | - |
| ODN-1c | + | + | + | + | + | + |
| ODN-1d | + | + | + | + | + | + |
| ODN-2b | + | (+) | - | - | - | - |
| ODN-2c | + | + | + | + | (+) | - |
| ODN-2d | + | + | + | + | + | + |
| Table 7. [¹²⁵I]ODN was incubated in 40 µL blood freshly drawn from a rabbit for times indicated at 39°C. The procedure was otherwise similar to that in the legend to Table 6. | | | | | | |

**TABLE 2:**

| Affinity of (k_{D}) of Bioconjugate Aptamers | | |
|---|---|---|
| **ODN** | **Type** | **k**_{**D**} |
| ODN 1a | unmodified | 45 nM |
| ODN 1e | 3'-biotin | 55 nM |
| ODN 1f | 3'-streptavidin | 41 nM, 99 nM |
| | | |
| ODN 2a | unmodified | 26.5 nM |
| ODN 2e | 3'-biotin | 85 nM |
| ODN 2f | 3'-streptavidin | 89 nM, 110 nM |
| Table 2. Fluorometric assay of the binding k_{D}s of bioconjugate aptamers to human α thrombin with a fluorescein-PPACK-labeled active site. The emitted intensity (522 nm) was recorded before and after the addition of aptamer to a thrombin solution (100 nM). The incident wavelength was 492 nm. | | |

## Claims

1. A composition comprising:
(a) A DNA aptamer that binds to thrombin; and
(b) streptavidin that is complexed to said aptamer at the 3' end or both, the 5'and 3' ends.

2. The composition of claim 1, wherein said aptamer is derivatized at the 3' end or at both, the 5' and 3' ends with biotin and said complex is formed by biotin and streptavidin.

3. The composition of claims 1 or 2, wherein said composition is further labelled with a radioactive label.

4. The composition of claim 3, wherein said radioactive label is ¹²³I or ¹²⁵I.

5. Use of a DNA aptamer that binds to thrombin and streptavidin that is complexed to said aptamer at the 3' end or both, the 5' and 3 ends for the preparation of a diagnostic composition for imaging thrombus formation.

## Patentansprüche

1. Zusammensetzung umfassend:
(a) ein DNA-Aptamer, das Thrombin bindet; und
(b) Streptavidin, das mit dem Aptamer am 3'-Ende oder sowohl am 5'- als auch am 3'-Ende komplexiert ist.

2. Zusammensetzung nach Anspruch 1, wobei das Aptamer am 3'-Ende oder sowohl am 5'- als auch am 3'-Ende mit Biotin derivatisiert ist und der Komplex von Biotin und Streptavidin gebildet wird.

3. Zusammensetzung nach Anspruch 1 oder 2, wobei die Zusammensetzung des weiteren mit einer radioaktiven Markierung markiert ist.

4. Zusammensetzung nach Anspruch 3, wobei die radioaktive Markierung ¹²³I oder ¹²⁵I ist.

5. Verwendung eines DNA-Aptamers, das Thrombin bindet und von Streptavidin, das mit dem Aptamer am 3'-Ende oder sowohl am 5'- als auch am 3'-Ende komplexiert ist, für die Herstellung einer diagnostischen Zusammensetzung zum Sichtbarmachen von Thrombusbildung.

## Revendications

1. Composition comprenant:
(a) un aptamère ADN qui lie la thrombine; et
(b) de la streptavidine complexée au dit aptamère, à l'extrémité 3' ou aux deux extrémités 3' et 5'.

2. Composition selon la revendication 1, dans laquelle ledit aptamère est fonctionnalisé par de la biotine à l'extrémité 3' ou aux deux extrémités 3' et 5', et ledit complexe est formé par la biotine et la streptavidine.

3. Composition selon les revendications 1 ou 2, dans laquelle ladite composition est en outre marquée par un marqueur radioactif.

4. Composition selon la revendication 3, dans laquelle ledit marqueur radioactif est ¹²³I ou ¹²⁵I.

5. Utilisation d'un aptamère ADN qui lie la thrombine et de streptavidine, qui est complexée au dit aptamère à l'extrémité 3' ou aux deux extrémités 3' et 5', pour la préparation d'une composition diagnostique pour l'imagerie de la formation de thrombus.
